# EUROPEAN PATENT APPLICATION

(11) **EP 3 117 785 A1**
(43) Date of publication of application: **18.01.2017**
(21) Application number: 15761904.0
(22) Date of filing: 02.03.2015
(51) Int. Cl.: A61B 17/32, F04B 43/04

(54) **LIQUID SPRAY DEVICE AND SURGICAL DEVICE**

(30) Priority: 12.03.2014 JP 2014048519
(71) Applicant: Seiko Epson Corporation, Tokyo 160-8801 (JP)
(72) Inventor: NAKAYASHIKI, Atsushi, Sendai-shi Miyagi 980-8577 (JP); SATO, Motohiko, Sendai-shi Miyagi 980-8577 (JP); NAKAGAWA, Atsuhiro, Sendai-shi Miyagi 980-8577 (JP); TOMINAGA, Teiji, Sendai-shi Miyagi 980-8577 (JP); KOJIMA, Hideki, Suwa-shi Nagano 392-8502 (JP)
(74) Representative: Hoffmann Eitle
(86) International application number: PCT/JP2015/001084
(87) International publication number: WO 2015/136883

(57) **Abstract**

A liquid ejection device that suppresses scatter of the living tissue, such as cells excised, fragmentated, or otherwise separated by ejected pulsed flow is provided.

The liquid ejection device ejects, through a nozzle 4, first liquid 12 in the form of pulses supplied through an ejection tube 3 and includes a supply tube 23 , through which second liquid 31 is supplied to a region in the vicinity of the nozzle 4. The liquid ejection device further includes a suction port 15, which is located in the vicinity of the nozzle 4, and a suction pump 18, which is connected to the suction port 15 via a suction tube 14 and sucks the first liquid 12 and the second liquid 31.

## Description

### Technical Field

The present invention relates to a liquid ej ection device and a surgical apparatus.

### Background Art

A medical apparatus that applies ejected fluid to a diseased part for treatment is used. For example, Patent Literature 1 describes a liquid ejection device. According to Patent Literature 1, the liquid ejection device drives a piezoelectric element to increase and decrease the volume of a fluid chamber to which physiological saline or any other liquid is supplied. As a result, pulsed flow is formed in the fluid chamber, and the liquid ejection device ejects the pulsed flow of the physiological saline or any other liquid through an ejection tube.

The liquid ejection device is used, for example, as a knife for medical use and can eject the pulsed flow of the physiological saline or any other liquid toward a living body to excise, fragmentate, or otherwise separate the living body.

### Citation List

### Patent Literature

PTL 1: JP-A-2008-82202

### Summary of Invention

### Technical Problem

Kinetic energy of the pulsed flow acts on a living body site to which the pulsed flow has been applied. Splashes containing living tissue, such as excised, fragmentated, or otherwise separated cells, burst out. As a result, the living tissue, such as the excised, fragmentated, or otherwise separated cells and the ejected pulsed flow scatter and contaminate the field under surgery. It is therefore desired to provide a liquid ejection device that suppresses the scatter of the living tissue, such as cells excised, fragmentated, or otherwise separated by the ejected pulsed flow.

### Solution to Problem

The invention has been made to solve the problem described above and can be implemented in the following forms or application example:

### [Application example 1]

A liquid ejection device according to this application example is a liquid ejection device that ejects a first liquid in a form of pulses through a liquid ejection opening of an ejection tube, the liquid ejection device including a second liquid supply unit that supplies a second liquid through a supply tube to a region in a vicinity of the liquid ejection opening.

According to this application example, the first liquid is ejected in the form of pulses through the liquid ejection opening of the ejection tube. The form of pulses means flow of fluid the flow rate or flow speed of which cyclically or irregularly varies (pulsation). The pulsed flow includes intermittent flow that repeats the state in which the fluid flows and the state in which the fluid stops but is not necessarily intermittent flow because the flow rate or flow speed of the fluid only needs to cyclically or irregularly varies.

That is, ejection of the first liquid in the form of pulses or ejection of pulsed flow of the first liquid means ejection of the first liquid the flow rate or flow speed of which cyclically or irregularly varies. At a location where the pulsed flow hits, living tissue, such as cells, is excised or fragmentated. Supplying the second liquid to the liquid ejection opening (nozzle section) allows a state in which the liquid ejection opening (front end of nozzle) is immersed in the second liquid (water column) to be achieved or the liquid wall formed of the second liquid (water wall) to be formed around the liquid ejection opening.

The positional relationship between the second liquid and the liquid ejection opening causes splashes containing living tissue, such as the excised or fragmentated cells, to be decelerated by the second liquid. As a result, scatter of living tissue, such as the cells excised, fragmentated, or otherwise separated by the ejected first liquid, can be suppressed.

### [Application example 2]

The liquid ejection device according to the application example described above includes a liquid suction opening provided in a vicinity of the liquid ejection opening and a suction unit that is connected to the liquid suction opening via a suction tube and sucks the first liquid and the second liquid.

According to this application example, excess second liquid can be sucked through the liquid suction opening provided in the vicinity of the liquid ejection opening. A situation in which the field under surgery is submerged in water can therefore be avoided.

### [Application example 3]

In the liquid ejection device according to the application example described above, the ejection tube is inserted through the supply tube.

According to this application example, since the ejection tube is inserted through the supply tube, the second liquid can be uniformly supplied in all directions around the liquid ejection opening. In the liquid ejection device, a liquid wall formed of the second liquid (water wall) is therefore likely to be formed around the liquid ejection opening. Scatter of the living tissue excised, fragmentated, or otherwise separated by the ejected first liquid and liquid containing the ejected first liquid beyond the second liquid around the liquid ejection opening can therefore be suppressed. Further, the direction in which the second liquid is supplied through the supply tube is allowed to follow the direction in which the first liquid is ejected through the liquid ejection opening.

### [Application example 4]

In the liquid ejection device according to the application example described above, the ejection tube is inserted through the suction tube.

According to this application example, since the ejection tube is inserted through the suction tube, the liquid built up at the liquid ejection opening can be uniformly sucked. The state in which the liquid ejection opening (front end of nozzle) is immersed in the second liquid and is not relatively unbiased with respect thereto (water column) is achieved, or the liquid wall formed of the second liquid (water wall) is formed around the liquid ejection opening. The suction tube sucks excess second liquid around the liquid ejection opening in a relatively uniform manner. Therefore, the second liquid is caused to uniformly present around the liquid ejection opening, whereby scatter of the ejected liquid can be suppressed.

### [Application example 5]

The liquid ejection device according to the application example described above includes a liquid chamber that changes a volume thereof to produce pulsed flow of the first liquid, a volume varying unit that changes an amount of change in the volume of the liquid chamber, and a controller that controls a supply quantity of the second liquid suppled from the second liquid supply unit on a basis of the amount of change in the volume of the liquid chamber.

According to this application example, when the volume of the liquid chamber changes by a large amount, the pulsed flow flows at a high speed, and the quantity of the ejected first liquid also increases, whereby the degree of the scatter also increases. In this case, the quantity of the second liquid supplied from the second liquid supply unit is increased. Scatter of the living tissue excised, fragmentated, or otherwise separated by the ejected first liquid and liquid containing the ejected first liquid beyond the second liquid around the liquid ejection opening can therefore be suppressed. When the volume of the liquid chamber changes by a small amount, the pulsed flow flows at a low speed, and the quantity of the ejected first liquid also decreases, whereby the degree of the scatter also decreases. The scatter can therefore be suppressed even when the quantity of the second liquid supplied from the second liquid supply unit is reduced. As a result, the consumption of the second liquid can be reduced.

### [Application example 6]

In the liquid ejection device according to the application example described above, when the volume varying unit increases the amount of change in the volume of the liquid chamber, the controller increases the quantity of the second liquid supplied from the second liquid supply unit.

According to this application example, the liquid ejection device includes the controller, which controls the quantity of supplied second liquid. When the amount of change in the volume of the liquid chamber is set at an amount larger than a predetermined amount of change, the pulsed flow flows at a high speed, and the quantity of ejected first liquid increases, so that the degree of the scatter increases. Since the controller increases the supply quantity of the second liquid supplied from the second liquid supply unit, the scatter of the first liquid can be suppressed.

### [Application example 7]

In the liquid ejection device according to the application example described above, when the volume varying unit reduces the amount of change in the volume of the liquid chamber, the controller reduces the quantity of the second liquid supplied from the second liquid supply unit.

According to this application example, the liquid ejection device includes the controller, which controls the quantity of supplied second liquid. When the amount of change in the volume of the liquid chamber is reduced, the pulsed flow flows at a low speed, and the quantity of ejected first liquid decreases, so that the degree of the scatter decreases. The controller reduces the supply quantity of the second liquid supplied from the second liquid supply unit, but the scatter remains suppressed. The consumption of the second liquid can therefore be reduced.

### [Application example 8]

The liquid ejection device according to the application example described above includes a switch for switching action of the first liquid between ejection and no ejection and a controller that controls the second liquid supply unit in such a way that the second liquid supply unit supplies the second liquid when the first liquid is ejected and the second liquid supply unit does not supply the second liquid when the ejection of the first liquid is stopped.

According to this application example, when the ejection of the first liquid is stopped, the second liquid supply unit does not supply the second liquid. No second liquid is therefore supplied in vain when no first liquid is ejected. The consumption of the second liquid can therefore be reduced.

### [Application example 9]

The liquid ejection device according to the application example described above includes a distance detector that detects a distance from the liquid ejection opening to an object present in a direction in which the first liquid is ejected, and the controller controls the second liquid supply unit in such a way that when the distance detected with the distance detector is shorter than a predetermined distance, the second liquid supply unit supplies the second liquid.

According to this application example, the distance detector detects the distance between the liquid ejection opening and the object. When the distance detected with the distance detector is shorter than the predetermined distance, the second liquid supply unit supplies the second liquid. When the liquid ejection opening is close to the object, the state in which the liquid ejection opening (front end of nozzle) is immersed in the second liquid (water column) is likely to be achieved, or the liquid wall formed of the second liquid (water wall) is likely to be formed around the liquid ejection opening (front end of nozzle). The second liquid 31 supplied from the second liquid supply unit can suppress scatter of the living tissue excised, fragmentated, or otherwise separated by the ejected first liquid and liquid containing the ejected first liquid. The consumption of the second liquid can be reduced because the controller supplies the second liquid when the second liquid effectively works.

### [Application example 10]

The liquid ejection device according to the application example described above includes a distance detector that detects a distance from the liquid ejection opening to an object present in a direction in which the first liquid is ejected, and the controller controls the second liquid supply unit in such a way that when the distance detected with the distance detector is longer than a predetermined distance, the supply of the second liquid from the second liquid supply unit is stopped.

According to this application example, when the liquid ejection opening is far away from the object, the state in which the liquid ejection opening (front end of nozzle) is immersed in the second liquid (water column) is unlikely to be achieved, or the liquid wall formed of the second liquid (water wall) is unlikely to be formed around the liquid ejection opening. In this case, no second liquid is wasted because the supply of the liquid is stopped in accordance with the distance. The consumption of the second liquid can be reduced because the controller supplies the second liquid when the second liquid effectively works.

When the liquid ejection opening is far away from the object, it is also effective to notify a user of the liquid ejection device that, in addition to the stoppage of the supply of the second liquid, the supply of the second liquid has been stopped in the form of audio or any other form. Further, in addition to the stoppage of the supply of the second liquid, the ejection of the first liquid may be stopped. Moreover, when the supply of the first liquid is stopped, the user of the liquid ejection device may be notified that the supply of the first liquid has been stopped in the form of audio or any other form.

### [Application example 11]

In the liquid ejection device according to the application example described above, surface tension of the second liquid is greater than surface tension of the first liquid.

According to this application example, surface tension of the second liquid is greater than surface tension of the first liquid. Cohesive force of the second liquid therefore increases at the liquid ejection opening, and the state in which the liquid ejection opening is immersed in the second liquid (water column) is likely to be achieved or the liquid wall formed of the second liquid (water wall) is likely to be formed around the liquid ejection opening. As a result, scatter of the ejected first liquid can be readily suppressed.

### [Application example 12]

In the liquid ejection device according to the application example described above, the second liquid supply unit supplies the first liquid to the point in the vicinity of the liquid ejection opening.

According to this application example, the first liquid is ejected through the liquid ejection opening, and the first liquid is supplied through the supply tube. The first liquid and the second liquid may therefore not be prepared separately. As a result, no container is required for the second liquid, whereby the liquid ejection device can be a compact device.

### [Application example 13]

The liquid ejection device according to the application example described above includes an adjuster that adjusts a flow rate of at least one of the second liquid and the sucked liquid in such a way that a sum of a flow rate of the first liquid ejected through the liquid ejection opening and a flow rate of the second liquid supplied from the second liquid supply unit is greater than a flow rate of liquid sucked through the liquid suction opening.

According to this application example, the adjuster adjusts the flow rates of the first liquid, the second liquid, and the sucked liquid. When the sum of the flow rate of the first liquid ejected through the liquid ejection opening and the flow rate of second liquid supplied from the second liquid supply unit is greater than the flow rate of the liquid sucked through the liquid suction opening, the state in which the liquid ejection opening (front end of nozzle) is immersed in the second liquid (water column) is likely to be achieved, or the liquid wall formed of the second liquid (water wall) is likely to be formed around the liquid ejection opening. As a result, the suction quantity and the quantity of the supplied second quantity are adjusted, whereby the water column or the water wall can be readily formed.

### [Application example 14]

The liquid ejection device according to the application example described above, the second liquid supply unit supplies the second liquid at least at a supply quantity of 5 ml/minute.

According to this application example, the supply flow rate of the second liquid supplied from the second liquid supply unit is at least 5 ml/min. When the supply flow rate of the second liquid is at least 5 ml/min with suction quantity required to suck the excised or fragmentated living tissue ensured, the state in which the liquid ejection opening (front end of nozzle) is immersed in the second liquid (water column) is likely to be achieved, or the liquid wall formed of the second liquid (water wall) is likely to be formed around the liquid ejection opening (front end of nozzle). Scatter of the ejected first liquid and living cells can therefore be suppressed.

### [Application example 15]

A surgical apparatus according to this application example includes anyone of the liquid ejection devices described above and the first liquid is ejected toward living tissue for a surgical treatment.

According to this application example, the surgical apparatus uses the liquid ejection device. Scatter of the living tissue excised, fragmentated, or otherwise separated by the ejected first liquid and liquid containing the ejected first liquid can therefore be suppressed. As a result, a surgical treatment (such as incision, excision, and fragmentation) can be performed without contamination of the field under surgery with the excised, fragmentated, or otherwise separated living tissue or liquid containing the ejected first liquid.

### Brief Description of Drawings

[Fig. 1] Fig. 1 relates to a first embodiment. Fig. 1(a) is a block diagram showing the configuration of a liquid ejection device, and Fig. 1 (b) is a partial diagrammatic side view showing the structure of a nozzle of the liquid ejection device.
[Fig. 2] Figs. 2 (a) and 2 (b) are diagrammatic views for describing the behavior of liquid at the nozzle.
[Fig. 3] Fig. 3 (a) is a diagrammatic cross-sectional view showing the internal configurationof apulsationapplyingpart,
Fig. 3 (b) shows graphs illustrating the transition of the volume of a liquid chamber, and Fig. 3(c) shows a graph illustrating the relationship of the quantity of supplied second liquid with the amount of change in the volume of the liquid chamber.
[Fig. 4] Fig. 4 is an electrical control block diagram of the liquid ejection device.
[Fig. 5] Fig. 5 is a flowchart of a method for cutting a surface of a living body.
[Fig. 6] Fig. 6 is a flowchart of a supply quantity adjustment step.
[Fig. 7] Fig. 7 relates to a second embodiment. Fig. 7 (a) is a block diagram showing the configuration of a liquid ejection device, and Fig. 7(b) is a partial diagrammatic side view showing the structure of a nozzle of the liquid ejection device.
[Fig. 8] Fig. 8 is a diagrammatic view for describing the behavior of liquid at the nozzle.
[Fig. 9] Fig. 9 relates to a third embodiment. Fig. 9 (a) is a block diagram showing the configuration of a liquid ejection device, and Fig. 9(b) is a partial diagrammatic side view showing the structure of a nozzle of the liquid ejection device.
[Fig. 10] Fig. 10 is a diagrammatic view for describing the behavior of liquid at the nozzle.
[Fig. 11] Fig. 11 is a block diagram showing the configuration of a liquid ejection device according to a fourth embodiment.
[Fig. 12] Fig. 12 is a block diagram showing the configuration of a liquid ejection device according to a fifth embodiment.
[Fig. 13] Fig. 13 relates to a sixth embodiment. Fig. 13 (a) is a block diagram showing the configuration of a liquid ejection device, and Fig. 13 (b) is a partial diagrammatic side view showing the structure of a nozzle of the liquid ejection device.
[Fig. 14] Fig. 14 is a diagrammatic view for describing the behavior of a liquid at the nozzle.

### Description of Embodiments

In an embodiment of the invention, a characteristic liquid ejection device and a characteristic example of a method for cutting a living body by using the liquid ejection device will be described with reference to the drawings. The embodiment will be described below with reference to the drawings. Members in the drawings are so drawn at different scales on a member basis as to be large enough to be recognizable in the drawings.

### (First embodiment)

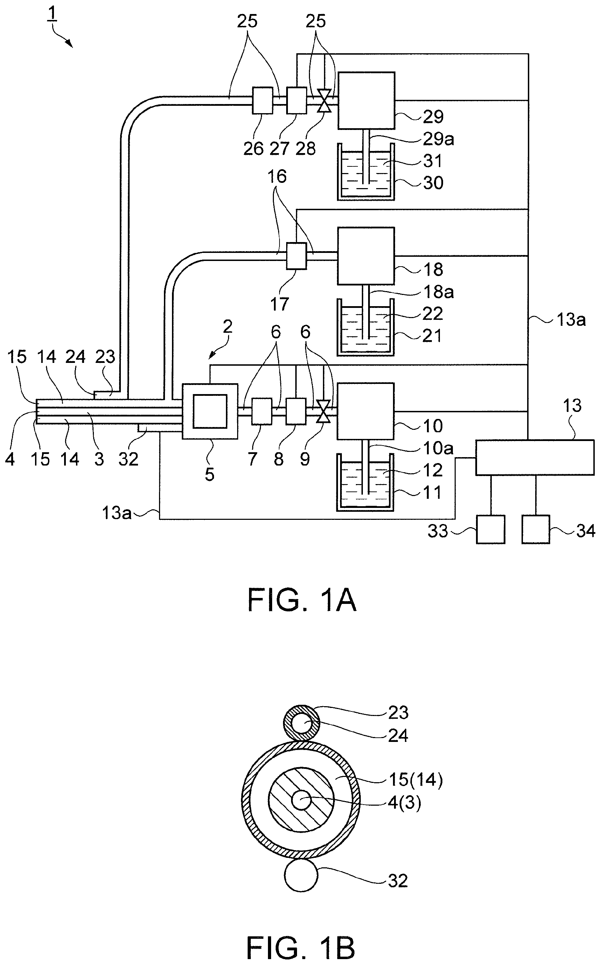

In the present embodiment, a liquid ejection device that is a surgical apparatus will be described with reference to Figs. 1 to 6. Fig. 1(a) is a block diagram showing the configuration of the liquid ejection device. Fig. 1(b) is a partial diagrammatic side view showing the structure of a nozzle of the liquid ejection device. A liquid ejection device 1 according to the present embodiment is a medical apparatus used in a medical institute and has a function of a knife for medical use that ejects fluid toward a patient to incise or excise a diseased part.

The liquid ejection device 1 includes a handpiece 2, as shown in Fig. 1(a). The handpiece 2 is an instrument gripped with a practitioner's hand and operated by the practitioner in surgery. The handpiece 2 is provided with an ejection tube 3, which is a channel through which the fluid flows. A nozzle 4, which serves as a liquid ejection opening through which the fluid is ejected, is provided at one end of the ejection tube 3. A pulsation applying part 5 is provided at the other end of the ejection tube 3. A first filter 7, a first flowmeter 8, a first electromagnetic valve 9, and a first pump 10 are connected in this order to the pulsation applying part 5 via a first tube 6. The pulsation applying part 5 is a section that converts the fluid passing therethrough into pulsed flow.

The first filter 7 has a function of removing foreign matter, bacteria, air bubbles, and other objects in the fluid. The first flowmeter 8 measures the flow rate of the fluid flowing through the first tube 6. The first flowmeter 8 can, for example, be a hot wire flowmeter or a turbine flowmeter. The first electromagnetic valve 9 is a valve so controlled with an electric signal as to open and close. The first electromagnetic valve 9 can be a valve that operates in such a way that a motor or an electromagnet opens and closes the valve.

The first pump 10 can be a syringe-type pump or a tube pump. When a syringe-type pump is used, it is preferable to provide a device that supplies the fluid into the syringe. The liquid ejection device 1 can thus be continuously driven.

The first pump 10 is provided with a water inlet tube 10a, and one end of the water inlet tube 10a is connected to a first water storage tank 11. The first water storage tank 11 stores a first liquid 12 as a first liquid. The first liquid 12 is, for example, physiological saline. Physiological saline, which does not harm a living body, can be used in surgical operation.

The liquid ejection device 1 includes a control device 13 as a controller, and the control device 13 controls the action of the liquid ejection device 1. The pulsation applying part 5, the first flowmeter 8, the first electromagnetic valve 9, and the first pump 10 are connected to the control device 13 via a cable 13a.

A suction tube 14 is provided in parallel to the ejection tube 3. A suction port 15, which serves as a liquid suction opening and a suction unit, is provided at the front end of the suction tube 14. The suction tube 14 opens at the suction port 15. The nozzle 4 and the suction port 15 are so positioned as to be roughly flush with each other, and the suction port 15 is disposed in the vicinity of the nozzle 4. A tube for suction 16 is connected to the suction tube 14. A suction flowmeter 17 and a suction pump 18 are connected in this order to the suction tube 14 via the tube for suction 16.

The suction flowmeter 17 can be a flowmeter of the same type as the first flowmeter 8. The suction pump 18 is not limited to a specific pump and can, for example, be a tube pump. The suction pump 18 is provided with a drain tube 18a, and the drain tube 18a is connected to a drain tank 21. The drain tank 21 stores drain 22 drained through the drain tube 18a. The suction flowmeter 17 and the suction pump 18 are connected to the control device 13 via the cable 13a. The suction port 15, the suction tube 14, the suction pump 18, and other components form the suction unit.

A supply tube 23 is provided in parallel to the ejection tube 3 and the suction tube 14. The supply tube 23 has a supply port 24 provided on the side facing the nozzle 4, and the supply tube 23 opens at the supply port 24. The supply port 24 is disposed in the vicinity of the nozzle 4. The supply tube 23 isprovidedwitha second tube 25 on the side facing the pulsation applying part 5. A second filter 26, a second flowmeter 27, a second electromagnetic valve 28, and a second pump 29 are connected in this order to the supply tube 23 via the second tube 25.

The second filter 26 can be a filter having the same function as that of the first filter 7 and of the same type as the first filter 7. The second flowmeter 27 can be a flowmeter having the same function as that of the first flowmeter 8 and of the same type as the first flowmeter 8. The second electromagnetic valve 28 can be a electromagnetic valve having the same function as that of the first electromagnetic valve 9 and of the same type as the first electromagnetic valve 9.

The second pump 29 is not limited to a specific pump and can, for example, be a tube pump. The second pump 29 is provided with a water inlet tube 29a, and one end of the water inlet tube 29a is connected to a second water storage tank 30. The second water storage tank 30 stores a second liquid 31 as a second liquid. The second liquid 31 is, for example, pure water. Pure water, which does not harm a living body, can be used in surgical operation. The second flowmeter 27, the second electromagnetic valve 28, and the second pump 29 are connected to the control device 13 via the cable 13a. The supply port 24, the supply tube 23, the second pump 29, and other components form a second liquid supplying unit.

Aproximity sensor 32, which serves as a distance detector, is provided on the side surface of the suction tube 14, and the proximity sensor 32 is connected to the control device 13 via the cable 13a. The handpiece 2 is used with the nozzle 4 located in the vicinity of a living body. The proximity sensor 32 measures the distance to the living body located in the vicinity of the nozzle 4. The proximity sensor 32 thus detects the distance between the nozzle 4 and the living body located in the direction in which the first liquid 12 is ejected through the nozzle 4. The proximity sensor 32 can be any of a variety of sensors, such as an electrostatic sensor, an optical sensor, and an ultrasonic sensor.

The control device 13 is provided with a main switch 33, an ejection switch 34, which serves as a switch, and other components. The main switch 33 is a switch that activates the liquid ejection device 1. When the main switch 33 is moved to the ON position, the control device 13 is energized. The ejection switch 34 is a switch that switches the fluid action through the nozzle 4 between ejection and no ejection. The ejection switch 34 is a switch operated by the practitioner's step-on action with a foot.

When the practitioner operates the main switch 33, the control device 13 is initialized. The practitioner then moves the ejection switch 34 to the ON position. The first pump 10 is activated and causes the first liquid 12 to flow to the first electromagnetic valve 9. When the control device 13 opens the first electromagnetic valve 9, the high-pressure first liquid 12 travels in the form of fluid through the first tube 6. The first flowmeter 8 detects the flow rate of the fluid traveling through the first tube 6 and outputs the flow rate to the control device 13.

The fluid traveling through the first tube 6 passes through the first filter 7. The first filter 7 removes dust, airbubbles, crystalline salt, and other objects from the first liquid 12. The first liquid 12 reaches the pulsation applying part 5, which applies pulsation to the first liquid 12. The first liquid 12 having passed through the pulsation applying part 5 passes through the ejection tube 3 and is ejected through the nozzle 4. Since pulsation had been applied to the first liquid 12 having passed through the nozzle 4, ejection in the form of pulses is achieved.

At the time when the practitioner moves the ejection switch 34 to the ON position, the suction pump 18 is activated concurrently with the activation of the first pump 10. The suction pump 18 sucks liquid located in the suction port 15. The sucked liquid enters the suction tube 14 through the suction port 15, passes through the tube for suction 16, and reaches the suction pump 18. The sucked liquid is then drained as the drain 22 into the drain tank 21. The suction flowmeter 17 detects the flow rate of the fluid traveling through the tube for suction 16 and outputs the flow rate to the control device 13.

At the time when the practitioner moves the ejection switch 34 to the ON position, the second pump 29 is activated concurrently with the activation of the first pump 10. When the second pump 29 is activated, the second pump 29 causes the second liquid 31 to flow to the second electromagnetic valve 28. When the control device 13 opens the second electromagnetic valve 28, the second liquid 31 travels in the form of fluid through the second tube 25. The second flowmeter 27 detects the flow rate of the fluid traveling through the second tube 25 and outputs the flow rate to the control device 13.

The fluid traveling through the second tube 25 passes through the second filter 26. The second filter 26 removes dust and other objects from the second liquid 31. The second liquid 31 having passed through the second tube 25 and the supply tube 23 and reached the supply port 25 is supplied to a space in the vicinity of the nozzle 4.

The first liquid 12 ejected through the nozzle 4 and the second liquid 31 supplied through the supply port 24 are partially sucked through the suction port 15.

The suction port 15 is disposed around the nozzle 4 concentrically therewith, and a structure in which the ejection tube 3 is inserted through the suction tube 14, as shown in Fig. 1(b). The liquid that builds up at the nozzle 4 can there fore be uniformly sucked. Therefore, the second liquid 31 is caused to uniformly present around the nozzle 4, whereby scatter of the ejected first liquid 12 can be suppressed.

The supply tube 23, which has a tubular shape, is disposed at the outer circumference of the suction tube 14. The proximity sensor 32 is disposed at the outer circumference of the suction tube 14 but on the side opposite the supply port 24 with the nozzle 4 therebetween. Since the proximity sensor 32 is set away from the supply port 24, an effect of the second liquid 31 supplied through the supply port 24 on the proximity sensor 32 can be reduced.

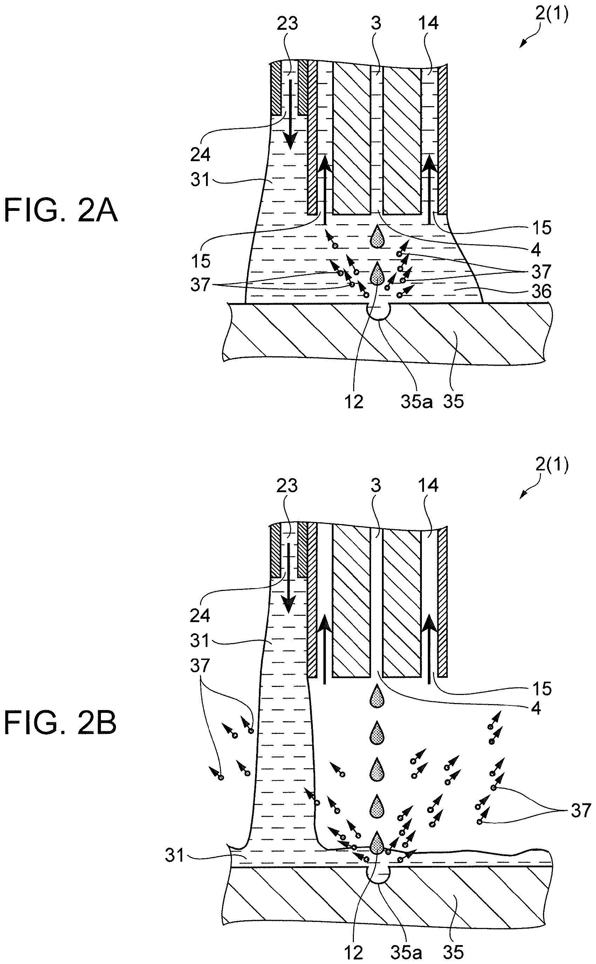

Figs. 2 (a) and 2 (b) are diagrammatic views for describing the behavior of the liquid at the nozzle. The practitioner operates the handpiece 2 to cause the nozzle 4 to approach a living body 35 as an object, as shown in Fig. 2(a). When the practitioner moves the ejection switch 34 to the ON position, the second liquid 31 is supplied through the supply port 24. The second liquid 31 then travels to the space between the nozzle 4 and the living body 35. Since surface tension is present on the second liquid 31, the second liquid 31 builds up in the space between the nozzle 4 and the living body 35 to form a liquid pool 36.

The second liquid 31 is pure water, and the first liquid 12 is physiological saline. The surface tension of the second liquid 31 is therefore greater than the surface tension of the first liquid 12. Cohesive force of the second liquid 31 therefore increases at the nozzle 4, and a state in which the nozzle 4 is immersed in the second liquid 31 (water column) is likely to be achieved or a liquid wall formed of the second liquid 31 (water wall) is likely to be formed around the nozzle 4. Fig. 2 (a) shows the water column. When a cavity is formed in the liquid pool 36 in the place facing the ejection tube 3, the liquid pool 36 has a tubular shape. The state is referred to as the water wall.

The control device 13 controls the flow rate of the second liquid 31 supplied through the supply port 24 and the flow rate of the liquid sucked through the suction port 15 in such a way that the flow rate of the sucked liquid is smaller than the flow rate of the second liquid 31 in the liquid pool 36. As a result, the liquid pool 36 is stably formed between the nozzle 4 and the living body 35.

The first liquid 12 ejected through the nozzle 4 hits the living body 35 in a hit point 35a. The first liquid 12 then separates a cell group 37, which is part of the living body 35, from the living body 35. The first liquid 12 having hit the living body 35 and the cell group 37 are decelerated when they move through the liquid pool 36. As a result, the first liquid 12 having hit the living body 35 and the cell group 37 build up in the liquid pool 36, whereby scatter of the first liquid 12 and the cell group 37 far away from the hit point 35a is suppressed.

The supply flow rate of the second liquid 31 supplied through the supply port 24 is set at 5 ml/minute or greater. When the supply flow rate of the second liquid 31 is 5 ml/minute or greater with the suction quantity required to suck excised or fragmentated cell group 37 ensured, the state in which the front end of the nozzle 4 is immersed in the second liquid 31 (water column) is likely to be achieved, or the liquid wall formed of the second liquid 31 (water wall) is likely to be formed around the nozzle 4. Scatter of the ejected first liquid 12 and the cell group 37 can therefore be suppressed.

When the nozzle 4 is separate from the living body 35 at least by a predetermined clearance, no water column or water wall is formed because the effect of gravity is greater than the effect of surface tension present on the second liquid 31, as shown in Fig. 2 (b). In this case, the first liquid 12 having hit the living body 35 and the cell group 37 scatter far away from the hit point 35a.

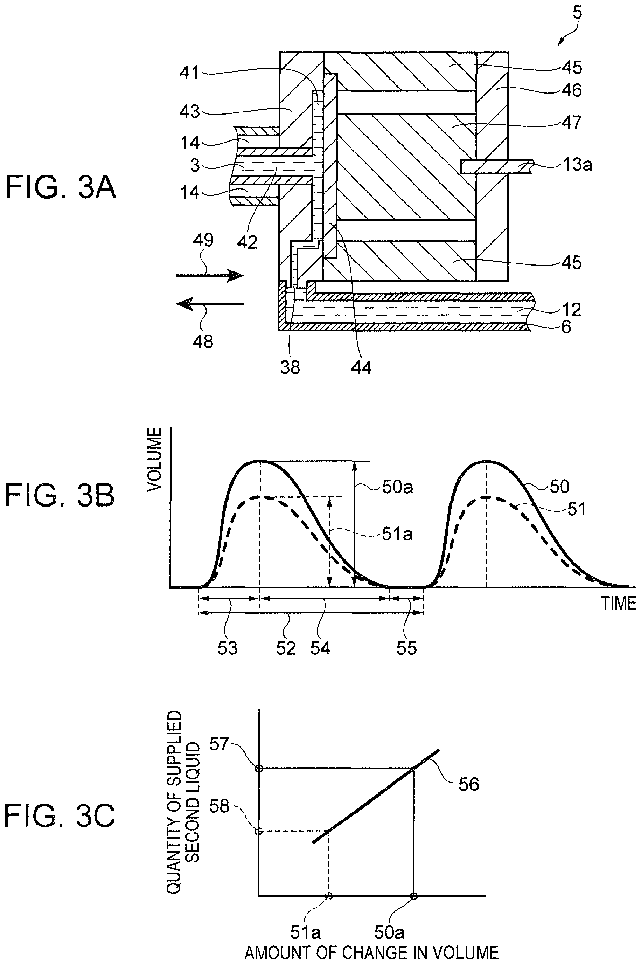

Fig. 3 (a) is a diagrammatic cross-sectional view showing the internal configuration of the pulsation applying part 5. The pulsation applying part 5 is provided with an inlet channel 38, a liquid chamber 41, and an outlet channel 42, through which the first liquid 12 supplied through the first tube 6 passes. The inlet channel 38 and the outlet channel 42 are formed in a first case 43. A diaphragm 44 is so provided that the first case 43 and the diaphragm 44 sandwich the liquid chamber 41. The first tube 6 is connected to the inlet channel 38, and the ejection tube 3 is connected to the outlet channel 42.

A tubular second case 45 is so provided as to be in contact with the first case 43 on the right thereof in Fig. 3 (a). The diaphragm 44 is a disk-shaped metal thin plate, and an outer circumferential portion of the diaphragm 44 is sandwiched and fixed between the first case 43 and the second case 45. A third case 46 is so provided as to be in contact with the second case 45 on the right thereof in Fig. 3(a). A piezoelectric element 47, which serves as a volume varying unit that is a laminated piezoelectric element, is provided between the diaphragm 44 and the third case 46. One end of the piezoelectric element 47 is fixed to the diaphragm 44, and the other end of the piezoelectric element 47 is fixed to the third case 46. The piezoelectric element 47 is connected to the control device 13 via the cable 13a.

When the control device 13 applies drive voltage to the piezoelectric element 47, the piezoelectric element 47 changes the volume of the liquid chamber 41, which is formed between the diaphragm 44 and the first case 43. When the drive voltage applied to the piezoelectric element 47 increases, the piezoelectric element 47 extends and presses the diaphragm 44, which then bends toward the liquid chamber 41 in a first direction 48 in Fig. 3(a). When the diaphragm 44 bends in the first direction 48, the volume of the liquid chamber 41 decreases. The fluid in the liquid chamber 41 is then pushed out of the liquid chamber 41. The inner diameter of the outlet channel 42 is greater than the inner diameter of the inlet channel 38. That is, the fluid resistance in the outlet channel 42 is smaller than the fluid resistance in the inlet channel 38. Further, since the inlet channel 38 is closer to the first pump 10 than the outlet channel 42, the water pressure in the inlet channel 38 is higher than the water pressure in the outlet channel 42. Most of the fluid in the liquid chamber 41 is therefore pushed out of the liquid chamber 41 through the outlet channel 42.

On the other hand, when the drive voltage applied to the piezoelectric element 47 decreases, the piezoelectric element 47 contracts and pulls the diaphragm 44, which then bends toward the third case 46 in a second direction 49 in Fig. 3(a). The piezoelectric element 47 contacts to increase the volume of the liquid chamber 41, and the fluid is supplied through the inlet channel 38 into the liquid chamber 41.

The drive voltage applied to the piezoelectric element 47 is repeatedly changed from ON (maximum voltage) to OFF (0 V) and vice versa at a high frequency (300 Hz, for example), so that the volume of the liquid chamber 41 repeatedly increases and decreases, whereby pulsation is applied to the fluid. The fluid pushed out of the liquid chamber 41 is ejected through the nozzle 4 at the front end of the ejection tube 3.

Fig. 3(b) shows graphs illustrating the transition of the volume of the liquid chamber. In Fig. 3(b), the vertical axis represents the volume, and the volume increases along the vertical axis from below to above in the figure. The horizontal axis represents transition of time, and the time transitions from left to right in the figure. A first transition line 50 represents the transition of the volume of the liquid chamber 41 in a case where the volume is change by a large amount. A second transition line 51 represents the transition of the volume of the liquid chamber 41 in a case where the volume is change by a small amount.

Each of the first transition line 50 and the second transition line 51 is repeated in the same cycle 52. The first transition line 50 and the second transition line 51 have similar shapes, and the transition of the change in the volume will therefore be described with reference to the first transition line 50. One cycle 52 is divided into a rising segment 53, a falling segment 54, and a pause segment 55. In the rising segment 53, the first transition line 50 has a shape similar to a sine waveform. During the segment, voltage is applied to the piezoelectric element 47, and the piezoelectric element 47 therefore extends. As a result, the diaphragm 44 moves in the first direction 48, and the volume of the liquid chamber 41 therefore decreases. The first liquid 12 in the liquid chamber 41 then moves into the outlet channel 42.

In the falling segment 54, the first transition line 50 has a shape similar to a sine waveform. During the segment, voltage applied to the piezoelectric element 47 decreases, and the piezoelectric element 47 therefore contracts. As a result, the diaphragm 44 moves in the second direction 49, and the volume of the liquid chamber 41 therefore increases. The first liquid 12 then flows through the inlet channel 38 into the liquid chamber 41. The falling segment 54 is longer than the rising segment 53. The first liquid 12 therefore bursts out to the outlet channel 42 and flows in through the inlet channel 38 at a low speed. The pause segment 55 is a segment where the piezoelectric element 47 maintains its contracting state. The cycle 52 can be adjusted by changing the length of the pause segment 55.

Now, let a first amount of change 50a be the amount of change in the volume along the first transition line 50 and a second amount of change 51a be the amount of change in the volume along the second transition line 51. The amount of change, such as the first amount of change 50a and the second amount of change 51a, can be adjusted by the piezoelectric element 47, which is controlled by the control device 13.

Fig. 3(c) shows a graph illustrating the relationship of the quantity of supplied second liquid with the amount of change in the volume of the liquid chamber. In Fig. 3(c), the vertical axis represents the supply quantity of second liquid 31 supplied through the supply port 24. The quantity increases along the vertical axis from below to above in the figure. The horizontal axis represents the amount of change in the volume of the liquid chamber 41, and the amount of change increases along the horizontal axis from left to right in the figure.

A volume-supply quantity correlation line 56 represents the relationship between the amount of change in the volume of the liquid chamber 41 and the quantity of supplied second liquid31. InFig. 3(c), thevolume-supplyquantitycorrelation line 56 is a straight line and may instead be a curve or a zigzag line. Let a first supply quantity 57 be the quantity of supplied second liquid 31 in the case where the amount of change in the volume of the liquid chamber 41 is the first amount of change 50a. Similarly, let a second supply quantity 58 be the quantity of supplied second liquid 31 in the case where the amount of change in the volume of the liquid chamber 41 is the second amount of change 51a.

Since the first amount of change 50a is greater than the second amount of change 51a, the volume of the pulsed flow of the first liquid 12 discharged through the nozzle 4 changes by a large amount. The larger the change in the volume of the pulsed flow, the greater the energy of the first liquid 12 that moves after it hits the living body 35. The first liquid 12 is therefore likely to scatter far away from the hit point 35a.

On the other hand, the first supply quantity 57, which corresponds to the first amount of change 50a, is greater than the second supply quantity 58, which corresponds to the second amount of change 51a. Therefore, even when a large amount of energy is applied to the pulsed flow of the first liquid 12, the volume of the liquid pool 36 increases because a large amount of second liquid 31 is supplied. Scatter of the first liquid 12 from the liquid pool 36 can therefore be suppressed.

On the other hand, when the control device 13 reduces the amount of change in the volume of the liquid chamber 41 from the first amount of change 50a to the second amount of change 51a, the control device 13 reduces the quantity of second liquid 31 supplied through the supply port 24 from the first supply quantity 57 to the second supply quantity 58. When the amount of change in the volume of the liquid chamber 41 is reduced, the pulsed flow of the ejected first liquid 12 flows at a low speed and the quantity of ejected first liquid 12 also decreases, whereby the degree of scatter decreases. The control device 13 reduces the supply quantity of second liquid 31 supplied through the supply port 24, but the scatter remains suppressed. The consumption of the second liquid 31 can therefore be reduced.

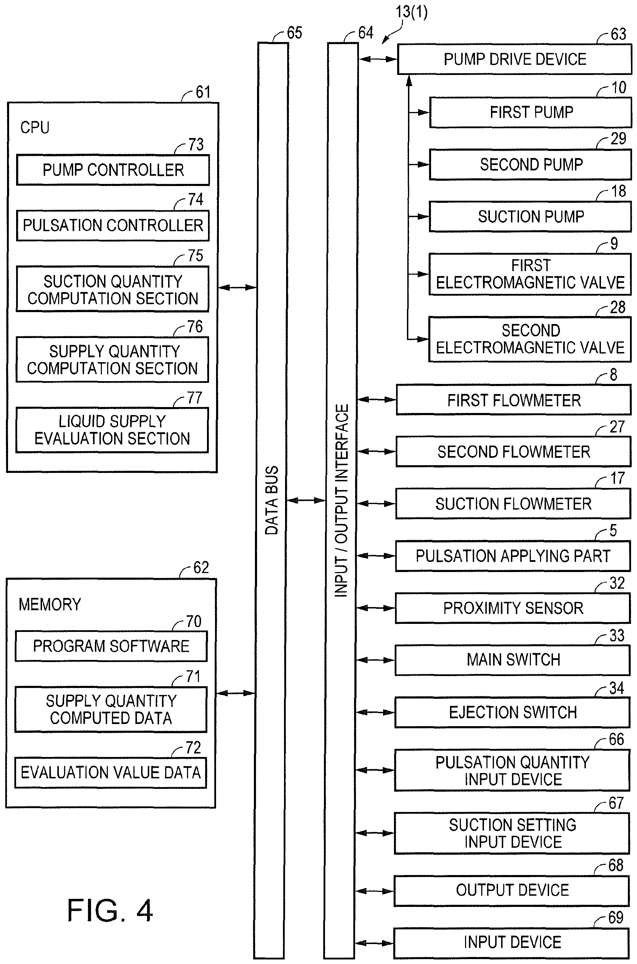

Fig. 4 is an electrical control block diagram of the liquid ejection device 1. In Fig. 4, the liquid ejection device 1 includes the control device 13, which controls the action of the liquid ejection device 1. The control device 13 includes a CPU 61 (central processing unit), which performs, as a processor, a variety of types of computation, and a memory 62, which stores a variety of pieces of information. A pump drive device 63, the first flowmeter 8, the second flowmeter 27, the suction flowmeter 17, the pulsation applying part 5, and the proximity sensor 32 are connected to the CPU 61 via an input/output interface 64 and a data bus 65. Further, the main switch 33, the ejection switch 34, a pulsation quantity input device 66, a suction setting input device 67, an output device 68, and in input device 69 are also connected to the CPU 61 via the input/output interface 64 and the data bus 65.

The pump drive device 63 is a device that drives the first pump 10, the second pump 29, the suction pump 18, the first electromagnetic valve 9, and the second electromagnetic valve 28. The pump drive device 63 receives, as an input, an instruction signal from the CPU 61. The pump drive device 63 drives the first pump 10, the second pump 29, and the suction pump 18 at the pressure or flow rate indicated by the instruction signal. Further, the pump drive device 63 drives the first electromagnetic valve 9 and the second electromagnetic valve 28 to open and close the valves.

The pulsation quantity input device 66 is a device to which the practitioner inputs the amount of change in the pulsation of the first liquid 12. The pulsation quantity input device 66 is, for example, a device for setting the amount of change in the volume of the liquid chamber 41, for example, to be the first amount of change 50a or the second amount of change 51a. The pulsation quantity input device 66 can be formed, for example, of a variable resistor and a circuit that converts the resistance of the variable resistor into voltage or any other circuit or a plurality of switches or other components.

The suction setting input device 67 is a device to which the practitioner sets the suction quantity of the liquid sucked through the suction port 15. The output device 68 includes a liquid crystal display device as well as a light or a loudspeaker that notifies malfunction, a device that performs wired or wireless communication with an external computer, and other components. The control device 13 can therefore output the state of the liquid ejection device 1 and the setting set by the practitioner.

The input device 69 includes a keyboard, a mouse-type input device, a pen-type input device as well as a device that performs wired or wireless communication with the external computer. The input device 69 inputs a variety of data to the memory 62.

The memory 62 is a concept including a semiconductor memory, such as a RAM and a ROM, and an external storage device, such as a hard disk drive and a DVD-ROM. In a functional sense, a storage area for storing program software 70, in which a control procedure of the action of the liquid ejection device 1 is written, and a storage area for storing supply quantity computed data 71, which is data used when the quantity of supplied second liquid 31 is computed, are set in the memory 62. In addition, a storage area for storing evaluation value data 72, which is data used to make evaluation when a variety of types of control are performed, is set in the memory 62. Still additionally, a storage area that functions, for example, as a work area and a temporary file for the CPU 61 and a variety of other storage areas are set in the memory 62.

The CPU 61 controls the handpiece 2 to cause it to eject the first liquid 12 through the nozzle 4 in accordance with the program software 72 stored in the memory 62. The CPU 61 has a pump controller 73 as a specific function achievement section. The pump controller 73 outputs an instruction signal to the pump drive device 63 to drive the first pump 10, the second pump 29, and the suction pump 18 so that the first liquid 12 and the second liquid 31 are caused to flow and sucked. Further, the pump controller 73 opens and closes the first electromagnetic valve 9 and the second electromagnetic valve 28 to start and stop the flow of the first liquid 12 and the second liquid 31.

The CPU further has a pulsation controller 74. The pulsation controller 74 receives, as an input, the pulsation quantity set by the pulsation quantity input device 66. The pulsation controller 74 then controls the piezoelectric element 47 in the pulsation applying part 5 to control the amount of change in the volume of the liquid chamber 41.

The CPU 61 further has a suction quantity computation section 75 as an adjuster. The suction quantity computation section 75 computes an appropriate suction quantity in accordance with the quantity of supplied second liquid 31. The suction quantity computation section 75 computes a total liquid flow rate that is the sum of the flow rate of the first liquid 12 ejected through the nozzle 4 and the flow rate of the second liquid 31 supplied through the supply port 24. The suction quantity computation section 75 then computes the suction quantity that is the flow rate of the liquid sucked through the suction port 15 in such a way that the flow rate of the sucked liquid is smaller than the total liquid flow rate. The CPU 61 then outputs the computed suction quantity to the pump controller 73.

When the sum of the flow rate of the first liquid ejected through the nozzle 4 and the flow rate of the second liquid 31 supplied through the supply port 24 is greater than the suction quantity sucked through the suction port 15, the state in which the front end of the nozzle 4 is immersed in the second liquid 31 (water column) is likely to be achieved, or the liquid wall formed of the second liquid 31 (water wall) is likely to be formed around the nozzle 4. Since the suction quantity is adjusted, scatter of the ejected first liquid 12 can be reduced.

The CPU 61 still further has a supply quantity computation section 76 as the adjuster. The supply quantity computation section 76 uses the volume-supply quantity correlation line 56 to compute the supply quantity of the second liquid 31 supplied through the supply port 24 on the basis of the amount of change in the volume of the liquid chamber 41. The supply quantity computation section 76 then output the computed supply quantity to the pump controller 73. The supply quantity computation section 76 and the pump controller 73 control the supply quantity.

When the volume of the liquid chamber 41 changes by a large amount, the pulsed flow of the first liquid 12 flows at a high speed, and the quantity of the ejected first liquid 12 also increases, whereby the degree of the scatter also increases. In this case, the quantity of the second liquid 31 supplied through the supply port 24 is increased. Scatter of the cell group 37 excised, fragmentated, or otherwise separated by the ejected first liquid 12 and liquid containing the ejected first liquid 12 can be suppressed. When the volume of the liquid chamber 41 changes by a small amount, the pulsed flow of the first liquid 12 flows at a low speed, and the quantity of the ejected first liquid 12 also decreases, whereby the degree of the scatter also decreases. The scatter can therefore be suppressed even when the quantity of the second liquid 31 supplied through the supply port 24 is reduced. As a result, the consumption of the second liquid 31 can be reduced.

The CPU 61 still further has a liquid supply evaluation section 77. The liquid supply evaluation section 77 drives the proximity sensor 32 to cause it to detect a nozzle-living body distance, which is the distance between the nozzle 4 and the living body 35. The liquid supply evaluation section 77 then compares the nozzle-living body distance with an evaluation value. When the nozzle-living body distance is smaller than the evaluation value, the liquid supply evaluation section 77 allows the second liquid 31 to be supplied through the supply port 24. When the nozzle-living body distance is greater than the evaluation value, the liquid supply evaluation section 77 causes the supply of the second liquid 31 through the supply port 24 to be terminated.

When the nozzle 4 is close to the living body 35, the state in which the front end of the nozzle 4 is immersed in the second liquid 31 (water column) is likely to be achieved, or the liquid wall formed of the second liquid 31 (water wall) is likely to be formed. The second liquid 31 supplied through the supply port 24 can suppress scatter of the cell group 37 excised, fragmentated, or otherwise separated by the ejected first liquid 12 and liquid containing the ejected first liquid 12. The consumption of the second liquid 31 can be reduced because the liquid supply evaluation section 77 supplies the second liquid 31 only when the second liquid 31 effectively works.

In the present embodiment, each of the functions described above is achieved by use of the CPU 61 along with the program software. Instead, when each of the functions described above can be achieved without use of the CPU 61 but use of a single electronic circuit (hardware), such an electronic circuit can be used.

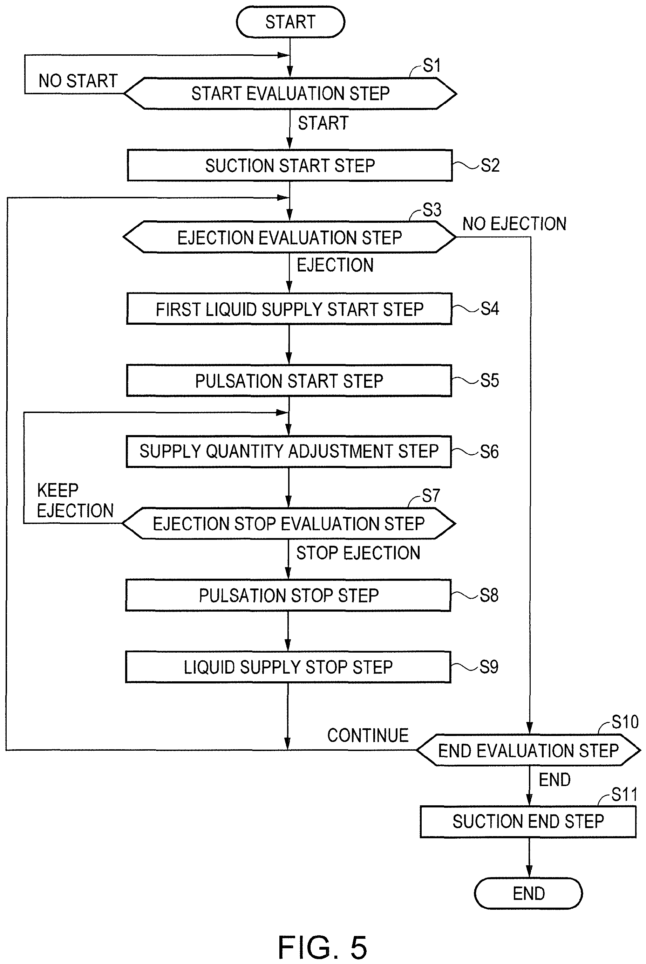

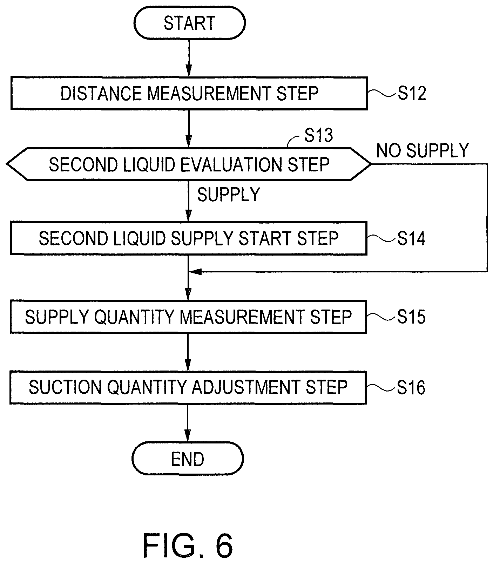

A method for cutting a surface of the living body 35 by using the liquid ejection device 1 described above will next be described with reference to Figs. 5 and 6. Fig. 5 is a flowchart of the method for cutting a surface of the living body, and Fig. 6 is a flowchart of a supply quantity adjustment step. In the flowchart of Fig. 5, step S1 corresponds to a start evaluation step. In this step S1, it is evaluated whether the practitioner has moved the main switch 33 to the ON position. A standby state continues until the practitioner moves the main switch 33 to the ON position, and step S1 then transitions to step S2 when the practitioner moves the main switch 33 to the ON position.

Step S2 corresponds to a suction start step. In this step, the pump controller 73 causes the pump drive device 63 to drive the suction pump 18. Since no second liquid 31 is supplied at this point, the suction pump 18 sucks air. Step S2 then transitions to step S3.

Step S3 corresponds to an ejection evaluation step. In this step, the CPU 61 detects whether the position of the ejection switch 34 is the ON or OFF position. When the position of the ejection switch 34 is the ON position, the CPU 61 determines that the first liquid 12 should be ejected, and step S3 then transitions to step S4. When the position of the ejection switch 34 is the OFF position, the CPU 61 determines that the first liquid 12 should not be ejected, and step S3 then transitions to step S10.

Step S4 corresponds to a first liquid supply start step. In this step, the pump controller 73 causes the pump drive device 63 to drive the first pump 10. The first pump 10 causes the first liquid 12 to flow toward the pulsation applying part 5. Step S4 then transitions to step S5.

Step S5 corresponds to a pulsation start step. In this step, the pulsation controller 74 receives, as an input, the pulsation setting quantity set by the pulsation quantity input device 66. The pulsation quantity is set in advance by the practitioner. The practitioner can also change the pulsation quantity in the course of the practice. The pulsation controller 74 drives the pulsation applying part 5 in such a way that the first liquid 12 is ejected in the set pulsation quantity. Step S5 then transitions to step S6.

Step S6 corresponds to a supply quantity adjustment step. Inthisstep, the supply quantity computation sect ion 7 6 computes the supply quantity of the second liquid 31 supplied through the supply port 24. The pump controller 73 then causes the pump drive device 63 to drive the second pump 29. The pump controller 73 then causes the second liquid 31 to be supplied through the supply port 24 by the computed supply quantity. Steps S4 to S6 are carried out roughly at the same time. Therefore, when the first liquid 12 is ejected, the second pump 29 supplies the second liquid 31 through the supply port 24.

The practitioner causes the nozzle 4 to approach the living body 35. As a result, the first liquid 12 is ejected toward the living body 35, and the cell group 37 of the living body 35 is removed as part of the living body 35. The practitioner's removal of the cell group 37 at a predetermined location from the living body 35 results in cutting of the living body 35. Step S6 then transitions to step S7.

Step S7 corresponds to an ejection stop evaluation step. In this step, the CPU 61 detects whether the position of the ejection switch 34 is the ON or OFF position. When the position of the ejection switch 34 is the ON position, the CPU 61 determines that the first liquid 12 should remain ejected, and step S7 then transitions to step S6. When the position of the ejection switch 34 is the OFF position, the CPU 61 determines that the ejection of the first liquid 12 should be stopped, and step S7 then transitions to step S8.

Step S8 corresponds to a pulsation stop step. In this step, the pulsation controller 74 stops driving the pulsation applying part 5. Step S8 then transitions to step S9.

Step S9 corresponds to a liquid supply stop step. In this step, the pump controller 73 causes the pump drive device 63 to stop driving the first pump 10 and the second pump 29. As a result, the ejection of the first liquid 12 through the nozzle 4 is stopped, and the supply of the second liquid 31 through the supply port 24 is also stopped. Step S9 then transitions to step S3.

Steps S7 to S9 are carried out roughly at the same time. When the ejection of the first liquid 12 is stopped, the pump controller 73 controls the first pump 10 and the second pump 29 in such a way that no second liquid 31 is supplied through the supply port 24. No second liquid 31 is therefore supplied in vain when no first liquid 12 is ejected. The consumption of the second liquid 31 can therefore be reduced.

Step S10 corresponds to an end evaluation step. In this step, it is evaluated whether the practitioner has moved the main switch 33 to the OFF position. When the practitioner keeps the main switch 33 in the ON position, it is determined that the practice continues and should not be stopped. Step S10 then transitions to step S3. When the practitioner moves the main switch 33 to the OFF position, it is determined that the practice has been completed. Step S10 then transitions to step S11.

Step S11 corresponds to a suction end step. In this step, the pump controller 73 causes the pump drive device 63 to stop driving the suction pump 18. After the steps described above are carried out, the step of cutting a surface of the living body ends.

Step S6, which is the supply quantity adjustment step, will next be described in detail with reference to Fig. 6. In Fig. 6, step S12 corresponds to a distance measurement step. In this step, the liquid supply evaluation section 77 drives the proximity sensor 32. The liquid supply evaluation section 77 then causes the proximity sensor 32 to detect the nozzle-living body distance, which is the distance between the nozzle 4 and the living body 35. Step S12 then transitions to step S13.

Step S13 corresponds to a second liquid evaluation step. In this step, the liquid supply evaluation section 77 compares the nozzle-living body distance with the evaluation value. When the nozzle-living body distance is smaller than the evaluation value, the liquid supply evaluation section 77 determines that the second liquid 31 should be supplied through the supply port 24. Step S13 then transitions to step S14. When the nozzle-living body distance is greater than the evaluation value, the liquid supply evaluation section 77 determines that the second liquid 31 should not be supplied through the supply port 24. The control device 13 may then drive the output device 68 to prompt the practitioner to cause the nozzle 4 to approach the living body 35. For example, the control device 13 may cause a loudspeaker to issue alarm sound or may display letters or a figure representing an alarm. Step S13 then transitions to step S15.

Step S14 corresponds to a second liquid supply start step. In this step, the pump controller 73 causes the pump drive device 63 to drive the second pump 29. The second pump 29 then causes the second liquid 31 to flow toward the supply port 24. Step S14 then transitions to step S15.

Step S15 corresponds to a supply quantity measurement step. In this step, the suction quantity computation section 75 drives the first flowmeter 8 and the second flowmeter 27. The first flowmeter 8 detects the flow rate of the first liquid 12 and outputs the flow rate to the suction quantity computation section 75. The second flowmeter 27 detects the flow rate of the second liquid 31 and outputs the flow rate to the suction quantity computation section 75. Step S15 then transitions to step S16.

Step S16 corresponds to a suction quantity adjustment step. In this step, the suction quantity computation section 75 computes the total liquid flow rate. The suction quantity computation section 75 computes a suction quantity indication value, which is the flow rate of the liquid sucked through the suction port 15 in such a way that the flow rate of the liquid to be sucked is smaller than the total liquid flow rate. The suction quantity computation section 75 then outputs the computed suction quantity indication value to the pump controller 73. The suction pump 18 adjusts the suction quantity through the suction port 15 in such a way that the suction quantity is equal to the suction quantity indication value.

The process of cutting the living body 35 is carried out concurrently with steps S12 to S16. The practitioner therefore ejects the first liquid 12 through the handpiece 2 to perform a surgical treatment on the living body 35. Step S6, which is the supply quantity adjustment step, has been described.

As described above, according to the present embodiment, the following advantageous effects are provided:
(1) According to the present embodiment, supplying the second liquid 31 through the supply port 24 to the nozzle 4 allows the state in which the front end of the nozzle 4 is immersed in the second liquid 31 (water column) to be achieved or the liquid wall formed of the second liquid 31 (water wall) to be formed around the nozzle 4. As a result, scatter of the cell group 37 excised, fragmentated, or otherwise separated by the ejected first liquid 12 and liquid containing the ejected first liquid 12 beyond the second liquid around the front end of the nozzle 4 can be suppressed.
(2) According to the present embodiment, supplying the second liquid 31 to the front end of the nozzle 4 allows the nozzle 4 to be immersed in the second liquid 31, whereby noise produced by vibration of the nozzle 4 can be absorbed and reduced by the second liquid 31.
(3) According to the present embodiment, excess second liquid 31 can be sucked through the suction port 15 disposed in the vicinity of the nozzle 4. A situation in which the field under surgery is submerged in water can therefore be avoided.
(4) According to the present embodiment, since the ejection tube 3 is inserted through the suction tube 14, the liquid built up at the nozzle 4 can be uniformly sucked. The state in which the front end of the nozzle 4 is immersed in the second liquid 31 and is not relatively unbiased with respect thereto (water column) is achieved, or the liquid wall formed of the second liquid 31 (water wall) is formed around the nozzle 4. Since the suction tube 14 sucks excess second liquid 31 around the nozzle 4 in a relatively uniform manner, scatter of the ejected first liquid 12 can be suppressed.
(5) According to the present embodiment, when the volume of the liquid chamber 41 changes by a large amount, the pulsed flow of the first liquid 12 flows at a high speed, and the first liquid 12 scatters by a large amount. In this case, the quantity of the second liquid 31 supplied through the supply port 24 is increased. Scatter of the cell group 37 excised, fragmentated, or otherwise separated by the ejected first liquid 12 and liquid containing the ejected first liquid 12 can therefore be suppressed. On the other hand, when the volume of the liquid chamber 41 changes by a small amount, the pulsed flow of the first liquid 12 flows at a low speed, and the quantity of the ejected first liquid 12 decreases, so that the degree of the scatter decreases. The scatter can therefore be suppressed even when the quantity of the second liquid 31 supplied through the supply port 24 is reduced. As a result, the consumption of the second liquid 31 can be reduced.
(6) According to the present embodiment, the liquid ejection device 1 includes the supply quantity computation section 76 and the pump controller 73, which control the quantity of supplied second liquid 31. When the volume of the liquid chamber 41 changes by a large amount, the pulsed flow of the first liquid 12 flows at a high speed, and the quantity of ejected first liquid 12 increases, so that the degree of the scatter increases. Since the supply quantity computation section 76 increases the supply quantity of the second liquid 31 supplied through the supply port 24, the scatter of the first liquid 12 can be suppressed.
(7) According to the present embodiment, when the volume of the liquid chamber 41 changes by a small amount, the pulsed flow of the first liquid 12 flows at a low speed, and the quantity of ejected first liquid 12 decreases, so that the degree of the scatter decreases. The supply quantity computation section 76 reduces the supply quantity of the second liquid 31 supplied through the supply port 24, but the scatter remains suppressed. The consumption of the second liquid 31 can therefore be reduced.
(8) According to the present embodiment, when the ejection of the first liquid 12 is stopped, no second liquid 31 is supplied through the supply port 24. No second liquid 31 is therefore supplied in vain when no first liquid 12 is ejected. The consumption of the second liquid 31 can therefore be reduced.
(9) According to the present embodiment, the proximity sensor 32 detects the distance between the nozzle 4 and the living body 35. When the distance detected with the proximity sensor 32 is shorter than the evaluation value, the liquid supply evaluation section 77 supplies the second liquid 31 through the supply port 24. When the nozzle 4 is close to the living body 35, the state in which the front end of the nozzle 4 is immersed in the second liquid 31 is likely to be achieved, or the liquid wall formed of the second liquid 31 is likely to be formed around the nozzle 4. The second liquid 31 supplied through the supply port 24 can suppress scatter of the cell group 37 excised, fragmentated, or otherwise separated by the ejected first liquid 12 and liquid containing the ejected first liquid 12. The consumptionof the second liquid 31 canbe reduced because the liquid supply evaluation section 77 supplies the second liquid 31 when the second liquid 31 effectively works.
(10) According to the present embodiment, when the nozzle 4 is far away from the living body 35, the state in which the front end of the nozzle 4 is immersed in the second liquid 31 (water column) is unlikely to be achieved, or the liquid wall formed of the second liquid 31 (water wall) is unlikely to be formed around the nozzle 4. In this case, the second liquid 31 supplied through the supply port 24 is wasted. When the nozzle 4 is far away from the living body 35, the supply of the second liquid 31 is stopped, whereby no second liquid 31 is wasted. The consumption of the second liquid 31 can therefore be reduced because the liquid supply evaluation section 77 supplies the second liquid 31 when the second liquid 31 effectively works.
(11) According to the present embodiment, the second liquid 31 is pure water, and the first liquid 12 is physiological saline. The surface tensionof the second liquid 31 is therefore greater than the surface tension of the first liquid 12. Cohesive force of the second liquid 31 therefore increases at the nozzle 4, and the state in which the nozzle 4 is immersed in the second liquid 31 (water column) is likely to be achieved or a liquid wall formed of the second liquid 31 (water wall) is likely to be formed around the nozzle 4. As a result, scatter of the ejected first liquid 12 can be readily suppressed.
(12) According to the present embodiment, the suction quantity computation section 75 adjusts the flow rate of the sucked liquid. When the sum of the flow rate of the first liquid 12 ejected through the nozzle 4 and the flow rate of second liquid 31 supplied through the supply port 24 is greater than the flow rate of the liquid sucked through the suction port 15, the state in which the front end of the nozzle 4 is immersed in the second liquid 31 (water column) is likely to be achieved, or the liquid wall formed of the second liquid 31 (water wall) is likely to be formed around the nozzle 4. Since the suction quantity computation section 75 adjusts the suction quantity, the water column or the water wall can be readily formed. As a result, scatter of the ejected first liquid 12 can be reduced.
(13) According to the present embodiment, the supply flow rate of the second liquid 31 supplied through the supply port 24 is at least 5 ml/min. When the supply flow rate of the second liquid 31 is at least 5 ml/min with suction quantity required to suck the excised or fragmentated cell group 37 ensured, the state in which the front end of the nozzle 4 is immersed in the second liquid 31 (water column) is likely to be achieved, or the liquid wall formed of the second liquid 31 (water wall) is likely to be formed around the nozzle 4. Scatter of the ejected first liquid 12 and the cell group 37 can therefore be suppressed.
(14) According to the present embodiment, the surgical apparatus uses the liquid ejection device 1. Scatter of the cell group 37 excised, fragmentated, or otherwise separated by the ejected first liquid 12 and liquid containing the ejected first liquid 12 can therefore be suppressed. As a result, a surgical treatment (such as incision, excision, and fragmentation) can be performed without contamination of the field under surgery with the excised, fragmentated, or otherwise separated cell group 37 or liquid containing the ejected first liquid 12.

### (Second embodiment)

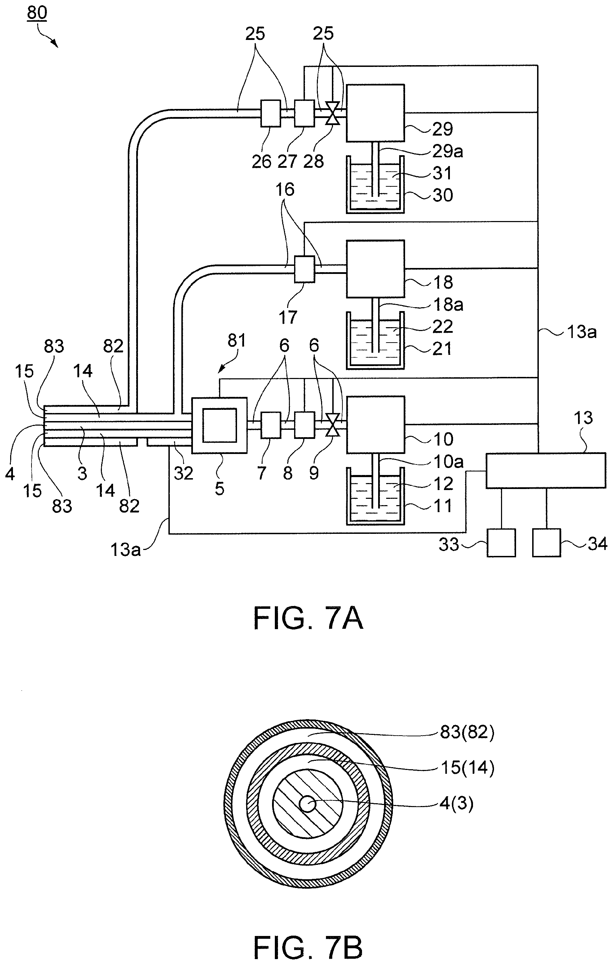

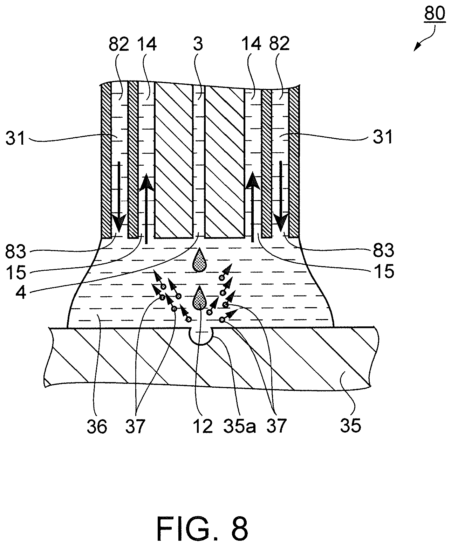

An embodiment of the liquid ejection device will next be described with reference to Figs. 7 and 8. Fig. 7(a) is a block diagram showing the configuration of the liquid ejection device. Fig. 7 (b) is a partial diagrammatic side view showing the structure of the nozzle of the liquid ejection device. Fig. 8 is a diagrammatic view for describing the behavior of liquid at the nozzle. The present embodiment differs from the first embodiment in that the suction tube 14 and the supply tube 23 shown in Fig. 1 are arranged differently. The same points as those in the first embodiment will not be described.

That is, in the present embodiment, a liquid ejection device 80 includes a handpiece 81, as shown in Fig. 7(a). The suction tube 14 is so provided as to surround the ejection tube 3, and a supply tube 82 is so provided as to surround the suction tube 14. A supply port 83 is provided at the front end of the supply tube 82. The nozzle 4, the suction port 15, and the supply port 83 are so disposed as to be flush with one another. The suction tube 14 and the supply tube 82 are so disposed as to be coaxial with the center of the ejection tube 3, as shown in Fig. 7 (b). That is, the ejection tube 3 is inserted through the supply tube 82.

The second liquid 31 supplied through the supply port 83 is sucked through the suction port 15 into the suction tube 14, as shown in Fig. 8. The liquid pool 36 is formed between the nozzle 4/suction port 15/supply port 83 and the living body 35. The first liquid 12 is then ejected through the nozzle 4 that is in the liquid pool 36 toward the hit point 35a.

As described above, according to the present embodiment, the following advantageous effects are provided:
(1) According to the present embodiment, since the ejection tube 3 is inserted through the supply tube 82, the liquid can be uniformly supplied in all directions around the nozzle 4. In the liquid ejection device 80, the liquid wall formed of the second liquid 31 (water wall) is therefore likely to be formed around the nozzle 4. Scatter of the cell group 37 excised, fragmentated, or otherwise separated by the ejected first liquid 12 and liquid containing the ejected first liquid 12 beyond the second liquid around the front end of the nozzle 4 can therefore be suppressed. Further, the direction in which the second liquid 31 is supplied through the supply tube 82 is allowed to follow the direction in which the first liquid 12 is ejected through the nozzle 4.

### (Third embodiment)

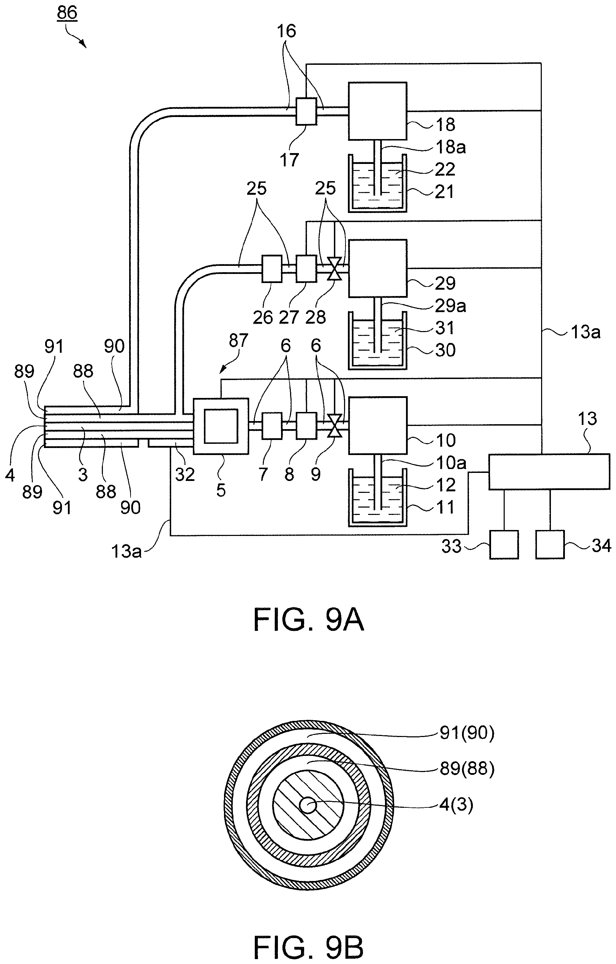

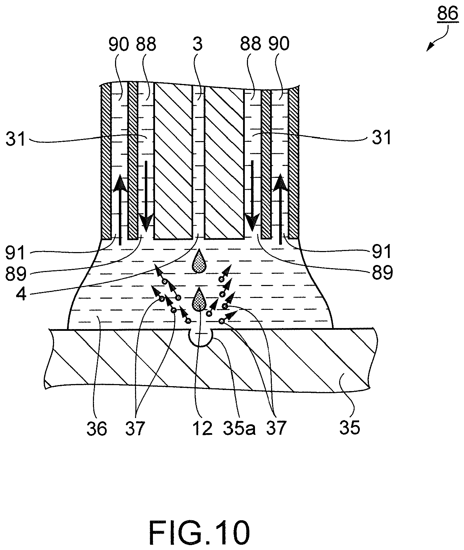

An embodiment of the liquid ejection device will next be described with reference to Figs. 9 and 10. Fig. 9(a) is a block diagram showing the configuration of the liquid ejection device. Fig. 9(b) is a partial diagrammatic side view showing the structure of the nozzle of the liquid ejection device. Fig. 10 is a diagrammatic view for describing the behavior of liquid at the nozzle. The present embodiment differs from the first embodiment in that the suction tube 14 and the supply tube 23 shown in Fig. 1 are arranged differently. The same points as those in the first embodiment will not be described.

That is, in the present embodiment, a liquid ejection device 86 includes a handpiece 87, as shown in Fig. 9(a). A supply tube 88 is so provided as to surround the ejection tube 3, and a supply port 89 is provided at the front end of the supply tube 88. A suction tube 90 is so provided as to surround the supply tube 88, and a suction port 91 is provided at the front end of the suction tube 90. The nozzle 4, the supply port 89, and the suction port 91 are so disposed as to be flush with one another. The supply tube 88 and the suction tube 90 are so disposed as to be coaxial with the center of the ejection tube 3, as shown in Fig. 9(b). That is, the ejection tube 3 is inserted through the supply tube 88.

The second liquid 31 supplied through the supply port 89 is sucked through the suction port 91 into the suction tube 90, as shown in Fig. 10. The liquid pool 36 is formed between the nozzle 4/supply port 89/suction port 91 and the living body 35. The first liquid 12 is then ejected through the nozzle 4 that is in the liquid pool 36 toward the hit point 35a.

As described above, according to the present embodiment, the following advantageous effects are provided:
(1) According to the present embodiment, since the ejection tube 3 is inserted through the supply tube 88, the liquid can be uniformly supplied in all directions around the nozzle 4. In the liquid ejection device 86, the liquid wall formed of the second liquid 31 (water wall) is therefore likely to be formed around the nozzle 4. Scatter of the cell group 37 excised, fragmentated, or otherwise separated by the ejected first liquid 12 and liquid containing the ejected first liquid 12 beyond the second liquid 31 around the nozzle 4 can therefore be suppressed. Further, the direction in which the second liquid 31 is supplied through the supply tube 88 is allowed to follow the direction in which the first liquid 12 is ejected through the nozzle 4.

### (Fourth embodiment)

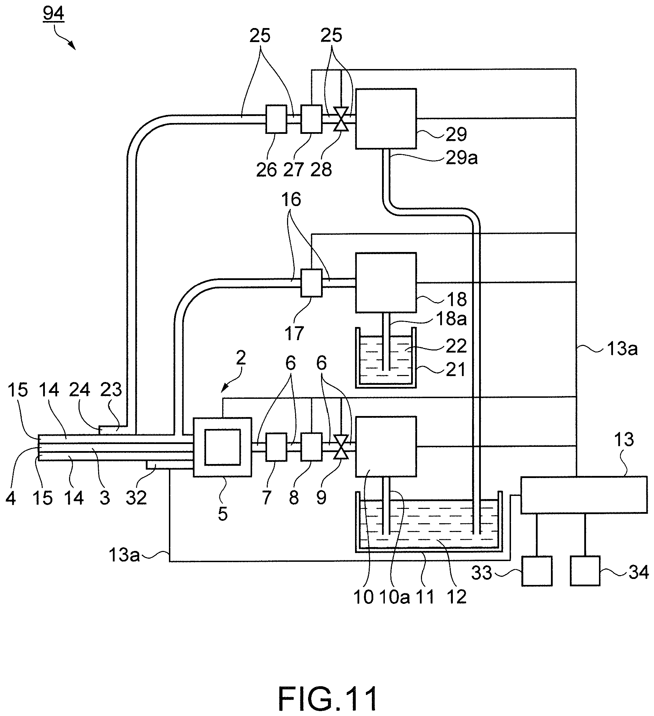

An embodiment of the liquid ejection device will next be described with reference to Fig. 11. Fig. 11 is a block diagram showing the configuration of the liquid ejection device. The present embodiment differs from the first embodiment in that the first pump 10 and the second pump 29 shown in Fig. 1 cause the same liquid to flow. The same points as those in the first embodiment will not be described.

That is, in a liquid ejection device 94 of the present embodiment, the first liquid 12 is stored in the first water storage tank 11, as shown in Fig. 11. The water inlet tube 10a of the first pump 10 and the water inlet tube 29a of the second pump 29 are connected to the first water storage tank 11. The first pump 10 and the second pump 29 therefore cause the first liquid 12 to flow. The first liquid 12 is then ejected through the nozzle 4 and also supplied through the supply port 24.

As described above, according to the present embodiment, the following advantageous effect is provided:
(1) According to the present embodiment, the first liquid 12 and the second liquid 31 may not be prepared separately. No container is therefore required for the second liquid 31, whereby the liquid ejection device 94 can be a compact device.

### (Fifth embodiment)

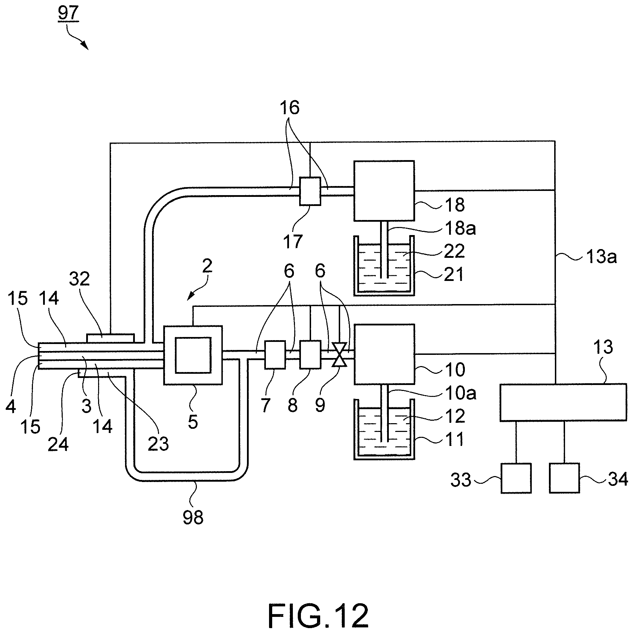

An embodiment of the liquid ejection device will next be described with reference to Fig. 12. Fig. 12 is a block diagram showing the configuration of the liquid ejection device. The present embodiment differs from the first embodiment in that the first pump 10 shown in Fig. 1 causes the same liquid to flow through the ejection tube 3 and the supply tube 23. The same points as those in the first embodiment will not be described.

That is, a liquid ejection device 97 of the present embodiment, a third tube 98 is connected to the first tube 6, which connects the first filter 7 to the pulsation applying part 5, as shown in Fig. 12. The third tube 98 then connects the first tube 6 to the supply tube 23.

The first liquid 12 caused to flow by the first pump 10 is supplied to the pulsation applying part 5 and the supply tube 23. The first pump 10 causes the first liquid 12 to flow. The first liquid 12 is then ejected through the nozzle 4 and also supplied through the supply port 24.

As described above, according to the present embodiment, the following advantageous effects are provided:
(1) According to the present embodiment, the first liquid 12 and the second liquid 31 may not be prepared separately. No container is therefore required for the second liquid 31, whereby the liquid ejection device 97 can be a compact device.
(2) According to the present embodiment, in the liquid ejection device 97, the first pump 10 causes the first liquid 12 to flow to the pulsation applying part 5 and the supply tube 23. The number of pumps can therefore be reduced as compared with a case where the first pump 10, which causes the first liquid 12 to flow to the pulsation applying part 5, and another pump that causes the first liquid 12 to flow to the supply tube 23 are provided. The liquid ejection device 97 can therefore be a compact device.

### (Sixth embodiment)

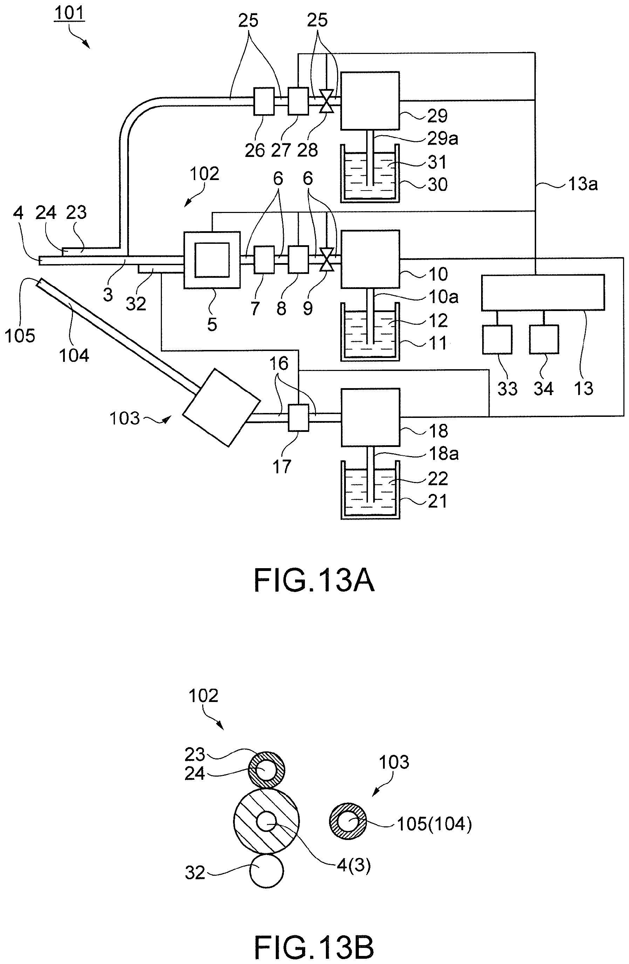

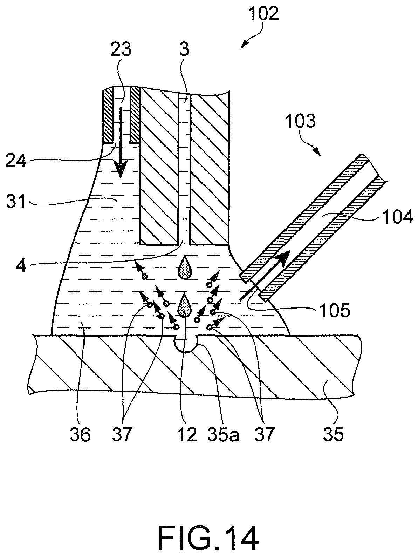

An embodiment of the liquid ejection device will next be described with reference to Figs. 13 and 14. Fig. 13(a) is a block diagram showing the configuration of the liquid ejection device. Fig. 13(b) is a partial diagrammatic side view showing the structure of the nozzle of the liquid ejection device. Fig. 14 is a diagrammatic view for describing the behavior of liquid at the nozzle. The present embodiment differs from the first embodiment in that the suction tube 14 shown in Fig. 1 is a member separate from the handpiece 2. The same points as those in the first embodiment will not be described.

That is, in the present embodiment, a liquid ejection device 101 includes a first handpiece 102 and a second handpiece 103, as shown in Fig. 13(a). The first handpiece 102 is provided with the pulsation applying part 5 and the ejection tube 3, which is connected to the pulsation applying part 5 is provided. The supply tube 23 and the proximity sensor 32 are disposed in parallel to the ejection tube 3.

The first liquid 12 caused to flow by the first pump 10 is supplied to the ejection tube 3. The pulsed flow of the first liquid 12 is then ejected through the nozzle 4. The second liquid 31 caused to flow by the second pump 29 is supplied to the supply tube 23. The second liquid 31 is then supplied through the supply port 24.

The second handpiece 103 includes a suction tube 104, and a suction port 105 is provided at the front end of the suction tube 104. The suction tube 104 is open at the suction port 105. The tube for suction 16 is connected to the suction tube 104. The suction flowmeter 17 and the suction pump 18 are connected to the suction tube 104 via the tube for suction 16.

The first handpiece 102 is provided with the nozzle 4 and the supply port 24, and the second handpiece 103 is provided with the suction port 105, as shown in Fig. 13(b). The practitioner can therefore separately operate the nozzle 4 and the suction port 105.

The practitioner operates the first handpiece 102 to cause the nozzle 4 to approach the living body 35, as shown in Fig. 14. When the practitioner moves the ejection switch 34 to the ON position, the second liquid 31 is supplied through the supply port 24. The second liquid 31 is then supplied to the space between the nozzle 4 and the living body 35. Since surface tension is present on the second liquid 31, the second liquid 31 builds up between the nozzle 4 and the living body 35 to form the liquid pool 36.

The practitioner or an assistant operates the handpiece 103 to insert the suction port 105 into the liquid pool 36. The liquid and the cell group 37 located in the liquid pool 36 are sucked through the suction port 105. The control device 13 controls the flow rate of the second liquid 31 supplied through the supply port 24 and the flow rate of the liquid sucked through the suction port 105 in such a way that the flow rate of the sucked liquid is smaller than the flow rate of the second liquid 31 in the liquid pool 36. As a result, the liquid pool 36 is stably formed between the nozzle 4 and the living body 35.

As described above, according to the present embodiment, the following advantageous effects are provided:
(1) According to the present embodiment, the first handpiece 102, which is provided with the nozzle 4, and the second handpiece 103, which is provided with the suction port 105, are members separately from each other. The practitioner operates the first handpiece 102 to adjust the position of the nozzle 4. At this point, the assistant can operate the second handpiece 103 provided with the suction port 105. Therefore, the practitioner can concentrate on the adjustment of the position of the nozzle 4, and the assistant can concentrate on the adjustment of the position of the suctionport 105. Since a surface of the living body 35 has protrusions and indentations, the second liquid 31 flows into the indentations of the living body 35 in some cases. Even in this case, the assistant can operate the second handpiece 103 to readily suck excess second liquid 31.

The present embodiment is not limited to the embodiments described above, and a variety of changes and improvements can be made thereto by a person skilled in the art within the technical idea of the invention. Variation will be described below.

### (Variation 1)

In the first embodiment described above, the suction quantity computation section 75 computes and adjusts the suction quantity on the basis of the quantity of the ejected first liquid 12 and the quantity of the supplied second liquid 31. The suction quantity is not necessarily adjusted, and the suction quantity sucked through the suction port 15 may be fixed. In this case, the supplyquantity computation section 76 may compute the supply quantity of the second liquid 31 in such a way that the total liquid flow rate of the quantity of the ejected first liquid 12 and the quantity of the supplied second liquid 31 is greater than the suction quantity. The supply quantity computation section 76 may then adjust the flow rate of the first liquid 12 and the flow rate of the second liquid 31. Since the suction quantity is fixed, the suction quantity can be readily controlled.

### (Variation 2)

In the first embodiment described above, the supply quantity computation section 76 uses the volume-supply quantity correlation line 56 to compute the supply quantity of the second liquid 31, as shown in Fig. 3(c). The supply quantity computation section 76 may instead use an evaluation value in accordance with which the amount of change in the volume of the liquid chamber 41 is evaluated to compute the supply quantity of the second liquid 31. That is, when the amount of change in the volume of the liquid chamber 41 increases beyond the evaluation value, the supply quantity computation section 76 increases the supply quantity of the second liquid 31. Further, when the amount of change in the volume of the liquid chamber 41 decreases beyond the evaluation value, the supply quantity computation section 76 may decrease the supply quantity of the second liquid 31. Since the supply quantity is not controlled continuously but is controlled stepwise, the control can be readily performed. The number of evaluation values may be one or plural.

### (Variation 3)

In the first embodiment described above, the piezoelectric element 47 is caused to contract toapplypulsation to the first liquid 12. Instead, force produced by an electromagnet, electrostatic force, or any other drive force may be used to apply pulsation to the first liquid 12. Still instead, in place of the piezoelectric element, bubbles are produced in the first liquid 12 supplied into a fluid chamber having a predetermined volume to apply pulsation to the first liquid 12. Still instead, a highly productive structure may be used.

### (Variation 4)

In the first embodiment described above, the liquid ejection device 1 is used as a medical apparatus for cutting the living body 35. The liquid ejection device 1 may be used in other applications. For example, the liquid ejection device 1 may be used to process food materials, suchasmeat, vegetables, and bean curd, and a variety of structural objects made, for example, of wood and concrete. Also in these cases, scatter of an object being processed can be avoided.

### Reference Signs List

1: Liquid ejection device as surgical apparatus, 3: Ejection tube, 4: Nozzle as liquid ejection opening, 12: First liquid as first liquid, 13: Control device as controller, 14: Suction tube as suction unit, 15: Suction port as liquid suction opening and suction unit, 18: Suction pump as suction unit, 23: Supply tube as second liquid supplying unit, 24: Supply port as second liquid supplying unit, 29: Second pump as second liquid supplying unit, 31: Second liquid as second liquid, 32: Proximity sensor as distance detector, 34: Ejection switch as switch
35: Living body as object, 41: Liquid chamber, 47: Piezoelectric element as volume varying unit, 75: Suction quantity computation section as adjuster, 76: Supply quantity computation section as adjuster

## Claims

1. A liquid ejection device that ejects a first liquid in a form of pulses through a liquid ejection opening of an ejection tube,
the liquid ejection device comprising a second liquid supply unit that supplies a second liquid through a supply tube to a region in a vicinity of the liquid ejection opening.

2. The liquid ejection device according to claim 1,
wherein the liquid ejection device comprises:
a liquid suction opening provided in a vicinity of the liquid ejection opening; and
a suction unit that is connected to the liquid suction opening via a suction tube and sucks the first liquid and the second liquid.

3. The liquid ejection device according to claim 2,
wherein the ejection tube is inserted through the supply tube.

4. The liquid ejection device according to any one of claims 1 to 3,
wherein the ejection tube is inserted through the suction tube.

5. The liquid ejection device according to any one of claims 1 to 4,
wherein the liquid ejection device comprises:
a liquid chamber that changes a volume thereof to produce pulsed flow of the first liquid;
a volume varying unit that changes an amount of change in the volume of the liquid chamber; and
a controller that controls a supply quantity of the second liquid suppled from the second liquid supply unit on a basis of the amount of change in the volume of the liquid chamber.

6. The liquid ejection device according to claim 5,
wherein, when the volume varying unit increases the amount of change in the volume of the liquid chamber, the controller increases the quantity of the second liquid supplied from the second liquid supply unit.

7. The liquid ejection device according to claim 5 or 6,
wherein, when the volume varying unit reduces the amount of change in the volume of the liquid chamber, the controller reduces the quantity of the second liquid supplied from the second liquid supply unit.

8. The liquid ejection device according to any one of claims 1 to 7,
wherein the liquid ejection device comprises:
a switch for switching action of the first liquid between ejection and no ejection; and
a controller that controls the second liquid supply unit in such a way that the second liquid supply unit supplies the second liquid when the first liquid is ejected, and
the second liquid supply unit does not supply the second liquid when the ejection of the first liquid is stopped.

9. The liquid ejection device according to any one of claims 1 to 7,
wherein the liquid ejection device comprises a distance detector that detects a distance from the liquid ej ectionopening to an object present in a direction in which the first liquid is ejected, and
the controller controls the second liquid supply unit in such a way that when the distance detected with the distance detector is shorter than a predetermined distance, the second liquid supply unit supplies the second liquid.

10. The liquid ejection device according to any one of claims 1 to 7,
wherein the liquid ejection device comprises a distance detector that detects a distance from the liquidej ectionopening to an object present in a direction in which the first liquid is ejected, and
the controller controls the second liquid supply unit in such a way that when the distance detected with the distance detector is longer than a predetermined distance, the supply of the second liquid from the second liquid supply unit is stopped.

11. The liquid ejection device according to any one of claims 1 to 10,
wherein surface tension of the second liquid is greater than surface tension of the first liquid.

12. The liquid ejection device according to any one of claims 1 to 10,
wherein the second liquid supply unit supplies the first liquid instead of the second liquid to the point in the vicinity of the liquid ejection opening.

13. The liquid ejection device according to claim 2,
wherein the liquid ejection device comprises an adjuster that adjusts a flow rate of at least one of the second liquid and the sucked liquid in such a way that a sum of a flow rate of the first liquid ejected through the liquid ejection opening and a flow rate of the second liquid supplied from the second liquid supply unit is greater than a flow rate of liquid sucked through the liquid suction opening.

14. The liquid ejection device according to any one of claims 1 to 13,
wherein the second liquid supply unit supplies the second liquid at least at a supply quantity of 5 ml/minute.

15. A surgical apparatus comprising:
the liquid ejection device according to any one of claims 1 to 14, and
the first liquid is ejected toward living tissue for a surgical treatment.
